# EUROPEAN PATENT APPLICATION

(11) **EP 4 431 124 A1**
(43) Date of publication of application: **18.09.2024**
(21) Application number: 23162106.1
(22) Date of filing: 15.03.2023
(51) Int. Cl.: A61L 29/08, A61L 29/14, A61L 29/16

(54) **NANOGEL COMPRISING BIOACTIVE MOLECULES, THERAPEUTIC MOLECULES OR DRUGS TOGETHER WITH AN AMPHIPHILIC MOLECULE, PARTICULARLY A VITAMIN-E DERIVATIVE**

(71) Applicant: CMD-COAT SA, 4000 Liège (BE)
(72) Inventor: OURY, Cécile, B-4000 Liège (BE); LANCELLOTTI, Patrizio, B-4000 Liège (BE); AQIL, Abdelhafid, B-4000 Liège (BE); DITKOWSKI, Bartosz, B-4000 Liège (BE)
(74) Representative: Barker Brettell LLP

(57) **Abstract**

New nanogel comprising one or more bioactive molecule, therapeutic molecule or drug together with an amphiphilic molecule and method of preparation thereof.

Biomaterial implant, medical device or bioprosthesis coated with the nanogel and method of producing thereof.

## Description

### FIELD OF THE INVENTION

The present invention relates to nanogel comprising bioactive molecules, therapeutic molecules or drugs together with an amphiphilic molecule, particularly vitamin E derivative. The present invention also relates to a medical device, biomaterial implant or bioprosthesis coated with the nanogel, a method of making such nanogel and a method of coating the medical device, biomaterial implant or bioprosthesis, particularly a catheter.

Most medical devices, biomaterial implants or bioprosthesis raise biocompatibility issues. Importantly, implantation of foreign materials in blood vasculature activates the contact pathway of coagulation, which may lead to thrombotic complications. For example, surface roughness of a medical device, biomaterial implants or bioprosthesis is an important factor influencing thrombogenicity.

Medical devices, biomaterial implants or bioprosthesis may also become infected and treatment for such infections generally requires administration of antibiotics targeting the causative bacteria.

Moreover, the increasing resistance to antibiotics has increased the demand for antibiotics exhibiting both antibacterial efficacy and anti-antibiotic resistance. As this demand has not been satisfied by a conventional one-target-one molecule approach, other approaches are required as the multi-target antibiotics.

Nanogels are known in the art. WO2018/122318A1 describes a nanogel made of a first hydrophilic polymer or copolymer bearing catechol groups and crosslinked with a second hydrophilic polymer bearing one or more reactive moieties; the nanogels also comprise bioactive molecules, therapeutic molecules or drugs.

Such nanogels are anchored or attached directly onto the surface of a medical device, biomaterial implant or bioprosthesis and are able to release the bioactive molecules, therapeutic molecules or drugs. Nevertheless, a difficulty in using such nanogels is the loading of water-insoluble or hydrophobic bioactive molecules, therapeutic molecules or drugs resulting in an irregular release kinetic profile and a lower pharmacological efficiency.

Moreover, only low levels of hydrophobic bioactive molecules, therapeutic molecules or drugs are released from such nanogel after release initiation. Immediate pharmacological effect can therefore not be rapidly achieved, and bacterial adhesion to the medical device, biomaterial implant or bioprosthesis cannot be fully prevented.

### SUMMARY OF THE INVENTION

We have now found an improved nanogel composition comprising one or more bioactive molecules, therapeutic molecules or drugs together with an amphiphilic molecule preferably a vitamin-E derivative for use in the coating of medical device, biomaterial implants or bioprosthesis; particularly the coating of catheter.

The present invention surprisingly and advantageously provides more efficient loading of hydrophobic bioactive molecules, therapeutic molecules or drugs in nanogels and a steady, progressive and continuous release over time during weeks. The hydrophobic bioactive molecules, therapeutic molecules or drugs can be combined with a hydrophilic bioactive molecules, therapeutic molecules or drugs in such nanogels, and be released simultaneously.

The use of such new nanogel composition in coating medical device, biomaterial implants or bioprosthesis also provides improved medical device, biomaterial implants or bioprosthesis.

The medical device, biomaterial implants or bioprosthesis coated with the nanogel of the invention, advantageously provides a more homogeneous, hydrophilic and smooth surface and content uniformity, resulting in a more reproducible kinetic release of the hydrophobic bioactive molecules, therapeutic molecules or drugs from the coated medical device, biomaterial implants or bioprosthesis, and subsequent improved pharmacological efficiency.

Simultaneously, medical device, biomaterial implants or bioprosthesis coated with the nanogel of the invention, advantageously reduces and delay bacterial adhesion to the surface of the medical device, biomaterial implants or bioprosthesis, particularly when the nanogel comprises a combination of a bacteriostatic agent and an antivrulence or a bactericidal agent.

### DETAILED DESCRIPTION

The present invention relates to nanogels which provide for a larger cargo space which may be used to incorporate bioactive molecule, therapeutic molecule or drug encapsulated by Vitamin E derivatives. Such nanogels can be anchored or attached onto the surface of any biomaterial or medical device, be it metallic or polymeric, or on a bioprosthesis and thereby reduce or prevent infection and improve biocompatibility and hemocompatibility of transiently or permanently implanted materials to help maintain their functionality and increase their durability.

**According to a first aspect,** the present invention provides a nanogel made of a poly(methacrylamide)-bearing quinone groups of formula (1) 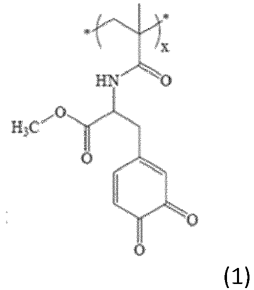
wherein x is an integer > 1, preferably x is between 1 and 100;
said poly(methacrylamide) been crosslinked with a polymer bearing primary or secondary amine groups; and
wherein the nanogel comprises one or more bioactive molecule, therapeutic molecule or drug together with an amphiphilic molecule, preferably a vitamin-E derivative, more preferably D-α-tocophenyl polyethylene glycol succinate.

Alternatively, the present invention also provides a nanogel made of a poly(vinylquinone) represented by formula (6) 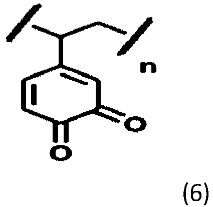
wherein n is an integer > 1; preferably n is between 1 and 100
said poly(vinylquinone) been crosslinked with a polymer bearing primary or secondary amine groups; and
wherein the nanogel comprises one or more bioactive molecule, therapeutic molecule or drug together with an amphiphilic molecule, preferably a vitamin-E derivative.

Alternatively, the present invention also provides a nanogel made of a combination of both quinone polymers, or copolymer made of poly(methacrylamide) and poly(vinylquinone). The present invention also extends to all polymers or copolymers bearing quinone groups.

The poly(methacrylamide)-bearing quinone groups of formula (1) is obtained by oxidation of catechol group (also known as benzene 1,2 diol group) of P(mDOPA) of formula (2). The oxidation is preferably carried out in aqueous media under basic conditions at pH above 10, preferably at a pH between 10 and 12.

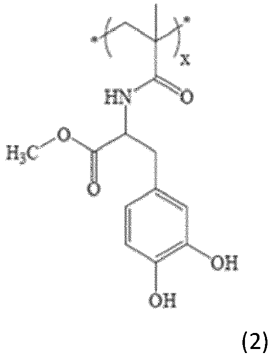

The poly(vinylquinone) of formula (6) is similarly obtained by oxidation of catechol group (also known as benzene 1,2 diol group) of poly(vinylcatechol) of formula (7) 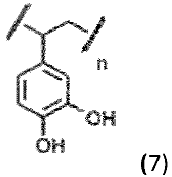 wherein n is an integer >1, preferably n is between 1 and 100.

The oxidation is preferably carried out in aqueous media under basic conditions, at pH above 10, preferably at a pH between 10 and 12.

The amphiphilic molecule is a molecule possessing both hydrophilic and lipophilic properties. Amphiphilic molecules have the property of self-assembly into micelles when dispersed in water. They may be a surfactant such as sodium dodecylsulfate, 1-octanol, cocamidopropyl betaine, benzalkonium chloride, phospholipids, cholesterol, glycolipids, fatty acids, a vitamin-polyether copolymer such as vitamin E- polyoxyalkylene copolymer, particularly D-α-tocopheryl polyethylene glycol succinate (TPGS).

The polymer bearing primary or secondary amine groups may be a polyallylamine, a polyvinylamine, a polyvinylamide, a polyvinylalcohol, a poly(meth)acrylate, a poly(meth)acrylamide, a polyurethane, a polyethylene glycol (PEG), a polyelectrolyte (cationic, anionic or zwitterionic) with reactive groups that are primary or secondary amines;

The polymer bearing primary or secondary amine groups may be a natural or a synthetic polymer with a primary or secondary amine function, for example polyvinyl amine, chitosan or a protein.

In a preferred embodiment, the polymer bearing primary amine groups may comprise a polyallylamine, such as poly-(allylamine hydrochloride) also known as PAH, as illustrated below wherein p is an integer >1, preferably p is between 10 and 300:

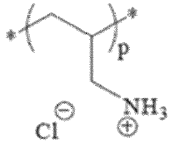

The bioactive molecule, therapeutic molecule or drug may be antibiotics, anti-biofilm formation agents, anti-platelet agents, anti-coagulants, anti-thrombotic agents, and anti-calcification agents.

Bioactive agents (also called bioactive molecules) may include any agent which is desired to be delivered to molecules, cells, tissues or organs for modulating or otherwise modifying molecule or cell function, including for therapeutic effects. Bioactive agents include, but are not limited to, pharmaceutically active compounds or diagnostic compounds. Bioactive compounds include, but are not limited to, nucleotides (aptamers, RNAi, antisense oligonucleotides), peptides, oligopeptides, proteins, apoproteins, glycoproteins, antigens and antibodies or antibody fragments thereto, receptors and other membrane proteins, retro-inverso oligopeptides, protein analogs in which at least one non-peptide linkage replaces a peptide linkage, enzymes, coenzymes, enzyme inhibitors, amino acids and their derivatives, hormones, lipids, phospholipids, liposomes, ricin or ricin fragments; toxins such as aflatoxin, digoxin, xanthotoxin, rubratoxin; analgesics such as aspirin, ibuprofen and acetaminophen; bronchodilators such as theophylline and albuterol; beta-blockers such as propranolol, metoprolol, atenolol, labetolol, timolol, penbutolol and pindolol; antimicrobial agents such as those described above and ciprofloxacin, cinoxacin and norfloxacin; antihypertensive agents such as clonidine, methyldopa, prazosin, verapamil, nifedipine, aptopril and enalapril; cardiovascular agents including antiarrhythmics, cardiac glycosides, antianginals and vasodilators; central nervous system agents including stimulants, psychotropics, antimanics and depressants; antiviral agents; antihistamines such as chlorphenirmine and brompheniramine; cancer drugs including chemotherapeutic agents, such as chlorambucil, carboplatin, derivatives of busulfan, doxorubicin, etoposide, topotecan (TPT); tranquilizers such as diazepam, chordiazepoxide, oxazepam, alprazolam and triazolam, anti-depressants such as fluoxetine, amitriptyline, nortriptyline and imipramine; H-2 antagonists such as nizatidine, cimetidine, famotidine and ranitidine; anticonvulsants; antinauseants; prostaglandins; muscle relaxants; anti-inflammatory substances; stimulants; decongestants; antiemetics; diuretics; antispasmodics; antiasthmatics; anti-Parkinson agents; expectorants; cough suppressants; mucolytics; vitamins; and mineral and nutritional additives. Other molecules include nucleotides; oligonucleotides; polynucleotides; and their art-recognized and biologically functional analogs and derivatives including, for example, methylated polynucleotides and nucleotide analogs having phosphorothioate linkages; plasmids, cosmids, artificial chromosomes, other nucleic acid vectors; antisense polynucleotides including those substantially complementary to at least one endogenous nucleic acid or those having sequences with a sense opposed to at least portions of selected viral or retroviral genomes; promoters; enhancers; inhibitors; other ligands for regulating gene transcription and translation.

The bioactive agent may be an anti-infective agent. Anti-infective agents include, but are not limited to antibiotics, such as amikacin, gentamicin, kanamycin, neomycin, netilmicin, tobramycin, paromomycin, streptomycin, spectinomycin, geldanamycin, herbimycin, rifaximin, loracarbef, ertapenem, dorpenem, imipenem/cilastatin, meropenem, cefadroxil, cefazolin, cefalotin, cephalexin, cefaclor, cefamandole, cefoxitin, cefproxil, cefuroxime, cefixime, cefdinir, cedfitoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, daibavancin, oritavancin, clindamycin, lincomycin, daptomycin, azithromycin, clarithromycin, dirithromycin, erythromycin, roxithromycin, troleandomycin, telithromycin, spiramycin, aztreonam, furazolidone, nitrofurantoin, linezolid, amoxicillin, ampicillin, piperacillin, ticarcillin, bacitracin, colistin, polymyxin B, ciprofloxacin, enoxacin, gatifloxacin, gemifloxacin, levoflaxicin, lomefloxacilin, moxifloxacin, nalidixic acid, norfloxacin, ofloxacin, mafenide, sulfacetamide, sulfadizine, silver sulfadizine, sulfadimethoxine, sulfamethizole, sulfamethoxazole, sulfanilimide, sulfisoxazole, trimethoprim-sulfamethoxazole, sulfonamidochrysoidine, demeclocyline, doxycycline, minocycline, oxytetracycline, tetracycline, clofazimine, dapsone, rifampicin, rifabutin, arspehnamine, chloramphenicol, fosfomycin, metronidazole, thiamphenicol, tigecycline, tinidazole, and trimethoprim.

Antibiotics may be either bacteriostatic to inhibit growth without killing or bactericidal for killing. The following antibiotics of the above-mentioned list are generally considered as bacteriostatic: minocycline (particularly for S. aureus), tetracyclines, macrolides, clindamycin, linezolid, chloramphenicol or a combination thereof, whereas the other antibiotics of the list above are most often bactericidal.

Antibiotics may also be antivirulence agents, when provided at low level.

Virulence factors are molecules produced by pathogens that allow colonization, immunoevasion, damaging host cells. Antivirulence agent targets virulence factors of pathogens instead of killing or stopping their growth, and consequently disarm infectious pathogens. In contrast to bactericidal antibiotics that drives resistance, antivirulence agents do not create a selective pressure driving resistance. Antivirulence agents interfere in interaction of the pathogen, particularly bacteria with host mammal, and thereby reduces damage to the host and impair bacteria ability to cause disease. Antivirulence agents can inhibit bacterial toxin production or prevent their adhesion to tissues.

Anti-biofilm formation agents include, but are not limited to naturally occurring peptides such as human cathelicidin LL-37 or the bovine peptide indolicidin, or synthetic peptides such as 1018, natural compounds with 2-aminoimidazole moiety, 2-aminoimidazole based inhibitors, benzimidazoles analogs, indole-triazo-amide analogs, plant-derived biofilm inhibitors such as emodin, phloretin, casbane diterpene, resveratrol and its oligomers, sulphur derivatives, brominated furanone analogs, bromopyrrole alkaloids, skyllamycins and (-)-ageloxime D structures, cembranoids, N-acyl homoserine lactone analogs, carolacton, molecules that interfere with the formation of amyloid-like fibres, fatty acids, nitric oxide donors, ionic liquids as 1-alkyl-3-methyl imidazolium chloride, 1-alkylquinolinium bromide, all these agents can be used in combination with conventional antibiotics.

Anti-platelet agents include, but are not limited to, irreversible cyclooxygenase inhibitors such as aspirin and triflusal (Disgren), adenosine diphosphate (ADP) receptor inhibitors such as clopidogrel (Plavix), prasugrel (Effient), ticagrelor (Brilinta), ticlopidine (Ticlid), Phosphodiesterase inhibitors such as cilostazol (Pletal), Protease-activated receptor-1 (PAR-1) antagonists such as vorapaxar (Zontivity), glycoprotein IIB/IIIA inhibitors (intravenous use only) such as abciximab (ReoPro), eptifibatide (Integrilin), tirofiban (Aggrastat), Adenosine reuptake inhibitors such as dipyridamole (Persantine), thromboxane inhibitors, thromboxane synthase inhibitors and thromboxane receptor antagonists such as terutroban, glycoprotein VI inhibitors such as Revacept, glycoprotein Ib inhibitors, and von Willebrand factor inhibitors.

Anti-coagulants include, but are not limited, to acenocoumarol, coumatetralyl, dicoumarol, ethyl biscoumacetate, phenprocoumon, warfarin, clorindione, dipjenadione, phenindione, ticlomarol, bemiparin, certoparin, ardeparin, dalteparin, enoxaparin, nadroparin, parnaparin, reviparin, dabigatran, apixaban, betrixabaan, darexaban, edoxaban, otamixaban, rivaroxaban, alteplase, danaparoid, tinzaparin, and fondaparinux.

Thrombolytic agents include, but are not limited to, alteplase, reteplase, tenecteplase, saruplase, urokinase, anistreplase, monteplase, streptokinase, ancrod, brinase and fibrinolysin.

Anti-calcification agents include, but are not limited to, bisphosphonates, aluminium salts, glutaraldehyde, amino oleic acid, and metalloproteinase inhibitors.

In a preferred embodiment, the nanogel according to the invention comprises an antibiotic and/or an antiplatelet agent.

In another preferred embodiment, the nanogel according to the invention comprises a combination of antibacterial agents, more preferably a bacteriostatic agent with an antivirulent agent inhibiting bacterial growth and bacterial adhesion when the nanogel is coated on a surface.

Advantageously, the nanogel comprising such combination of a bacteriostatic and antivirulence agents delays bacterial metabolic rate and increase anti-adhesive property when the nanogel is coated on a medical device, a biomaterial implant or bioprosthesis.

In a more preferred embodiment, the bacteriostatic agent is minocycline.

In another preferred embodiment, the bioactive molecule, therapeutic molecule or drug is a triazolo(4,4-d)-pyrimidine derivative of formula (3) 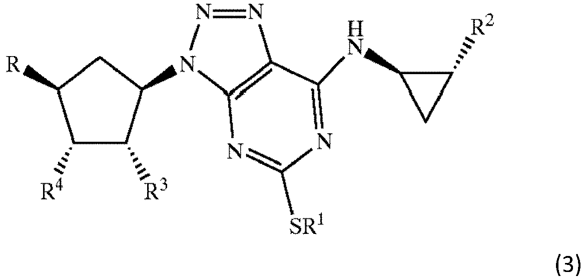 wherein R1 is C3-5 alkyl optionally substituted by one or more halogen atoms; R₂ is a phenyl group, optionally substituted by one or more halogen atoms; R₃ and R₄ are both hydroxyl; R is OH or XOH, wherein X is CH₂, OCH₂CH₂, or a bond;
or a pharmaceutical acceptable salt or solvate thereof, or a solvate thereof or a solvate of such a salt provided that when X is CH₂ or a bond, R₁ is not propyl; when X is CH₂ and R₁ CH₂CH₂CF₃, butyl or pentyl, the phenyl group at R₂ must be substituted by fluorine; when X is OCH₂CH₂ and R₁ is propyl, the phenyl group at R₂ must be substituted by fluorine.

The triazolo(4,4-d)-pyrimidine derivatives of formula (3) have advantageously an antiplatelet, but also an antibacterial effect. It is particularly useful to reduce or prevent infection of blood-contacting medical device, biomaterial implants or bioprosthesis, when inserted or implanted into a mammal host, and to prevent thrombosis. Thrombosis indeed promotes infection of blood-contacting medical device, biomaterial implants or bioprosthesis. The mammal host may be a human patient or an animal.

In a most preferred embodiment the triazolo (4,4-d)-pyrimidine derivative is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called Triafluocyl.

In another most preferred embodiment the triazolo (4,4-d)-pyrimidine derivative is (1S,2R,3S,4R)-4-[7-[[(1R,2S)-2-(3,4-Difluorophenyl)-cyclopropyl]amino]-5-(propylthio)-3H-1,2,3-triazolo[4,5-d]pyrimidin-3-yl]-1,2,3-cyclopentanetriol , also called Fluometacyl and illustrated in formula (5).

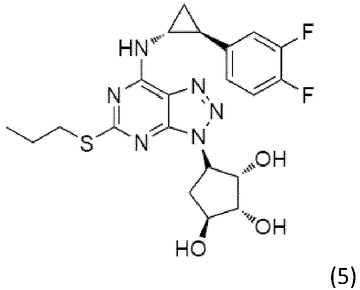

In another preferred embodiment, the bioactive molecule, therapeutic molecule or drug is a pyrimidine derivative represented by formula (4) 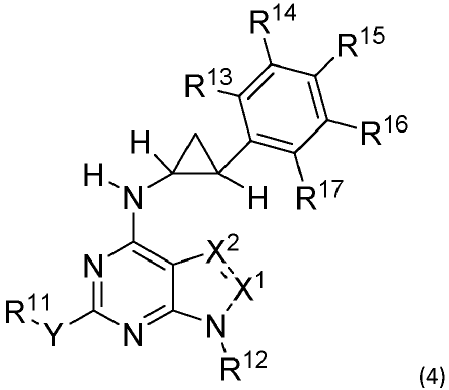 or optical isomers, racemic mixtures thereof, pharmaceutically acceptable acid addition salts, pharmaceutically acceptable metal salts, or alkylated ammonium salts or prodrug thereof;
wherein:
X¹ and X² are independently N, CH, CR⁸ wherein R⁸ is C ₁₋₆ alkyl, C ₂₋₆ alkenyl or C ₂₋₆ alkynyl ; with the exception that if one of X¹ or X² is equal to N, then the remaing X¹ or X² are selected from CH, CR⁸ .
-Y- is -O- or -S-;
R¹¹ and R¹² are independently C₁₋₆ -alkyl, C₂₋₆-alkenyl, C₂₋₆-alkynyl, C₃₋₆-cycloalkyl, aryl, aryl-C₁₋₆-alkyl wherein the alkyl or cycloalkyl moiety is optionally mono or polysubstituted with OH or an halogen and the aryl moiety is optionally mono or polysubstituted with an halogen, -C₁₋₆ alkyl, -C₁₋₆ alkoxy, -OH, -NO₂, -CN, -NH₂, - NHR⁸, -N(R⁸)₂ -COOH, -COOR⁸, -CONH₂, -CONHR⁸, -CON(R⁸)₂, -SO₂NH₂, -SO₂NHR⁸, or -SO₂N(R⁸)₂;
R¹³, R¹⁴, R¹⁵, R¹⁶ and R¹⁷ are independently H, an halogen, a C₁₋₆ alkyl, C₁₋₆ alkoxy, -OH, -NO₂, -CN, -NH₂, -NHR⁸, -N(R⁸)₂ -COOH, -COOR⁸, -CONH₂, -CONHR⁸, - CON(R⁸)₂, -SO₂NH₂, -SO₂NHR⁸, or -SO₂N(R⁸)₂.

The pyrimidine derivatives of formula (4) have advantageously an anti-bacterial effect. They are particularly useful to reduce or prevent infection of medical device, biomaterial implants or bioprosthesis when inserted or implanted into a mammal host. The mammal host may be a human patient or an animal.

The bioactive agent, therapeutic molecule or drug is dispersed in a solvent together with an amphiphilic molecule, preferably a vitamin-E derivative before addition into the resulting dispersion of poly(methacrylamide)-bearing quinone groups.

The solvent may be any solvent comprising O-H group. The solvent should not contain N-H bond to avoid an interaction with a catechol group. The solvent is for example water, alcohol such as methanol, ethanol, butanol, propanol and the like, or a combination thereof.

The vitamin-E derivative may be any copolymer obtained by esterification of Vitamin-E (also called α-tocopherol succinate) with an acid ester such as Vitamin E acetate or with a linear or branched polyether such as polyoxyalkylene as for example, polyoxyethylene, polyoxypropylene, polyoxypropylene-polyoxyethylene copolymer, polyethylene glycol, polypropylene glycol, and the like.

The polyalkylene glycol have a molecular weight between 500 and 2000, preferably between 750 and 1000, most preferably 1000.

In a preferred embodiment Vitamin-E derivative is
D- α-tocopheryl polyethylene glycol succinate (TPGS), represented in formula A. TPGS is a copolymer obtained by esterification with polyethylene glycol (PEG 750 or PEG1000) of Vitamin-E (also called α-tocopherol succinate)

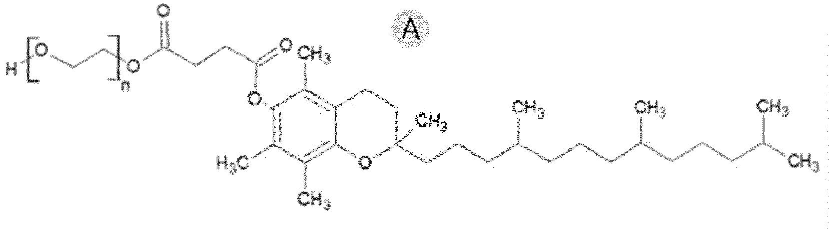

The amphiphilic molecule, particularly vitamin E derivatives, more particularly TPGS copolymer forms efficiently micelles into solvent such as water or an aqueous solution containing 0 to 60% of alcohol as for example ethanol. TPGS encapsulates hydrophobic bioactive agent, therapeutic molecule or drug and increase the loading of such hydrophobic bioactive agent, therapeutic molecule or drug into the nanogel, but also their efficiency and bioavailability by a continuous release from within the nanogel over weeks, preferably more than 10 days.

TPGS copolymer forms efficiently micelles with hydrophobic bioactive molecule, therapeutic molecule or drug into an aqueous solution; when mixed in a ratio TPGS: (bioactive agent, therapeutic molecule or drug) of 1:1 w/w to 5:1 w/w; preferably 1:1w/w.

The hydrophilic moiety of TPGS copolymer, polyethylene glycol (PEG), forms the corona of the micelles whereas the hydrophobic moiety, tocopherol succinate forms their core. The hydrophobic core of micelles can solubilize poorly soluble or insoluble drugs and partly protect the bioactive agent, therapeutic molecule or drug from aqueous environment. TPGS molecules encapsulate the hydrophobic bioactive agent, therapeutic molecule or drug and contribute to their better stability when inserted into the nanogel.

The micelles obtained by such encapsulation of the bioactive agent, therapeutic molecule or drug have a mean particles size in the range of 10 to 100nm, preferably 10nm.

The bioactive agent, therapeutic molecule or drug together with an amphiphilic molecule, preferably a TPGS, encapsulated by TPGS is entrapped as micelles within nanogels and/or between the nanogels.

The nanogel comprises nanoparticles with micelles of bioactive agent, therapeutic molecule or drug together with amphiphilic molecule, preferably TPGS. A nanoparticle is any particle wherein the longest dimension is less than 1000nm, e.g. about 100nm to 300nm. For example, the nanogel may have a longest dimension of less than about 500nm, less than about 300nm, less than about 200nm, less than about 150nm. In particular embodiments, the nanogel of the present invention comprises nanoparticles which have a diameter of about 150nm to about 250nm. In particular embodiments, the nanogel of the present invention comprises nanoparticles which have a diameter of about 100 to about 250nm.

When the micelles are loaded into the nanogel, the amphiphilic molecule, particularly TGPS surprisingly reduce bacterial adhesion to coated medical device, biomaterial implants or bioprosthesis and does not prevent the pharmacological effect of the bioactive agent, therapeutic molecule or drug as it can in a solvent mixture.

The nanogel according to the invention can advantageously load a higher level of bioactive molecule, therapeutic molecule or drug. Consequently, the nanogel according to the invention also advantageously allows a more prolonged release in contact with cells, tissues or organs when coated on medical device, biomaterial implants or bioprosthesis.

**According to a second aspect,** the present invention provides a method of making the nanogel comprising one or more bioactive molecules, therapeutic molecules or drugs, the method comprising the steps of:
i)mixing poly(methacrylamide)-bearing quinone groups of formula (1) 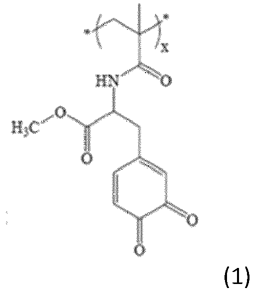
   wherein x is an integer > 1, preferably x is between 1 and 100
   with one or more bioactive molecules, therapeutic molecules or drugs together with an amphiphilic molecule, preferably vitamin-E derivative;
ii)adding a solution of polymer bearing primary or secondary amine groups to the resulting mixture obtained in step i) to generate nanogel comprising one or more bioactive molecule, therapeutic molecule or drug together with an amphiphilic molecule, preferably a vitamin-E derivative.

In the first step (i), micelle formation occurs directly after addition of a solution of poly(methacrylamide)-bearing quinone groups of formula (1) to the mixture of one or more bioactive molecules, therapeutic molecules or drugs together with an amphiphilic molecule, preferably a vitamin-E derivative in a solvent.. The solvent may be water or alcohol or a combination thereof.The solvent is preferably ethanol. The solution of poly(methacrylamide)-bearing quinone groups may be water or alcohol or a combination thereof, but is preferably water.

The addition is carried out under stirring at room temperature.

In the second step (ii), the polymer bearing primary or secondary amine groups react through a quinone-amine reaction, with poly(methacrylamide)- bearing quinone groups and of formula (1) to generate a nanogel comprising one or more bioactive molecules, therapeutic molecules or drugs with an amphiphilic molecule, preferably a vitamin-E derivative.

Alternatively, the present invention provides a method of making the nanogel comprising bioactive molecules, therapeutic molecules or drugs, the method comprising the steps of:
i)mixing a poly(vinyl)quinone of formula (6) 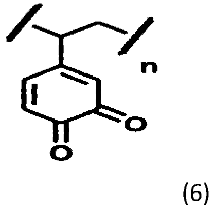
   wherein n is an integer > 1 preferably n is between 1 and 100;
   with one or more bioactive molecule, therapeutic molecule or drug together with an amphiphilic molecule, preferably a vitamin-E derivative;
ii)adding a solution of polymer bearing primary or secondary amine groups to the resulting mixture obtained in step i) to generate a crosslinked nanogel comprising one or more bioactive molecule, therapeutic molecule or drug together with an amphiphilic molecule, preferably vitamin-E derivative.

In the first step (i), micelle formation and encapsulation with the amphiphilic molecule occurs directly after addition of a solution of poly(vinyl)quinone of formula (6) to the mixture of one or more bioactive molecules, therapeutic molecules or drugs with an amphiphilic molecule, preferably a vitamin-E derivative in a solvent.

The solvent may be water or alcohol or a combination thereof, but is preferably ethanol.

The solution of poly(methacrylamide)-bearing quinone groups may be water or alcohol or a combination thereof, but is preferably water.

The addition is carried out under stirring at room temperature.

In the second step (ii), the polymer bearing primary or secondary amine groups react through a quinone-amine interaction, to the poly(vinylquinone) of formula (6) to generate nanogel comprising one or more bioactive molecule, therapeutic molecule or drug encapsulated with the amphiphilic molecule, preferably the vitamin-E derivative.

In a preferred embodiment, the vitamin-E derivative is D-α-tocophenyl polyethylene glycol succinate

In another preferred embodiment, the polymer bearing primary or secondary amine groups comprises a polyallylamine, preferably poly-(allylamine hydrochloride) also known as PAH, as illustrated below wherein p is an integer >1, preferably p is between 10 and 300, most preferably p is 160:

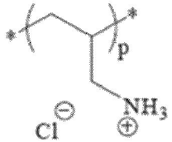

The solvent may be any solvent comprising a O-H group and therefore at least one hydrogen susceptible to interact in Hydrogen bonding. They are for example water, alcohol such as methanol, ethanol, butanol, propanol and the like, or a combination thereof.

In a preferred embodiment the solvent is an alcohol, preferably ethanol.

In another preferred embodiment the bioactive molecule, therapeutic molecule or drug is a triazolo(4,4-d)-pyrimidine derivative of formula (3), most preferably the triazolo(4,4-d)-pyrimidine derivative is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called triafluocyl or (1S,2R,3S,4R)-4-[7-[[(1R,2S)-2-(3,4-Difluorophenyl)-cyclopropyl]amino]-5-(propylthio)-3H-1,2,3-triazolo[4,5-d]pyrimidin-3-yl]-1,2,3-cyclopentanetriol also called Fluometacyl.

In another preferred embodiment the triazolo(4,4-d)-pyrimidine derivative, is combined with a bacteriostatic antibiotic, preferably minocycline. The triazolo(4,4-d)-pyrimidine derivative is preferably Triafluocyl or Fluometacyl.

**According to a third aspect,** the present invention provides a biomaterial implant, medical device or bioprosthesis wherein a surface or part thereof is coated with the nanogel made of a poly-(methacrylamide)-bearing quinone groups of formula (1) 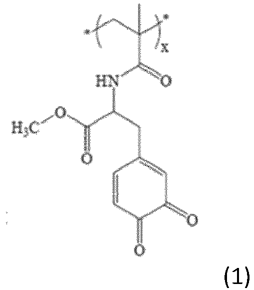
wherein x is an integer > 1, preferably in the range between 1 and 100
said poly-(methacrylamide) been crosslinked with a polymer bearing primary or secondary amine groups; and
wherein the nanogel comprises one or more bioactive molecule, therapeutic molecule or drug together with an amphiphilic molecule, preferably vitamin-E derivative, more preferably D-α-tocophenyl polyethylene glycol succinate.

Alternatively, the present invention provides a biomaterial implant, medical device or bioprosthesis wherein a surface or part thereof is coated with the nanogel made of a poly(vinyl)quinone of formula (6) 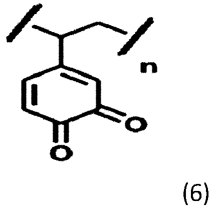
wherein n is an integer >1, preferably in the range between 1 and 100;
said poly(vinyl)quinone been crosslinked with a polymer bearing primary or secondary amine groups; and
wherein the nanogel comprises one or more bioactive molecule, therapeutic molecule or drug together with an amphiphilic molecule, preferably a vitamin-E derivative, more preferably D-α-tocophenyl polyethylene glycol succinate.

Alternatively, the present invention provides a biomaterial implant, medical device or bioprosthesis wherein a surface or part thereof is coated with the nanogel made of poly-(methacrylamide)-bearing quinone and polyvinylquinone or the copolymer thereof.

The present invention also extends to all biomaterial implant, medical device or bioprosthesis wherein a surface or part thereof is coated with the nanogel made of polymers or copolymers bearing quinone groups.

Biomaterial implant, medical device or bioprosthesis coated with the nanogel according to the invention advantageously provides a more homogeneous, hydrophilic and smooth surface

Such more homogeneous, hydrophilic and smooth surface of biomaterial implant, medical device or bioprosthesis coated with the nanogel advantageously reduces injuries, when inserted into human patient or animal.

Biomaterial implant, medical device or biosprosthesis coated with the nanogel according to the invention also advantageously provides a more reproducible kinetic release of the hydrophobic bioactive molecules, therapeutic molecules or drugs from the nanogel and from the medical device, biomaterial implants or bioprosthesis, resulting in a better pharmacological efficiency of bioactive molecules, therapeutic molecules or drugs, particularly to prevent infection and thrombotic complications.

Moreover, biomaterial implant, medical device or bioprosthesis coated with the nanogel according to the invention also advantageously provides reduced bacterial adhesion at their surface.

When the micelles comprising one or more bioactive molecule, therapeutic molecule or drug encapsulated with an amphiphilic molecule, preferably a vitamin-E derivative, more preferably D-α-tocophenyl polyethylene glycol succinate; are loaded into the nanogel coated on the surface of the Biomaterial implant, medical device or bioprosthesis; bacterial adhesion is reduced on the surface of the biomaterial implant, medical device or bioprosthesis. Such anti-adhesion effect is synergistically enhanced when a bacteriostatic agent is present into the nanogel

Moreover, the amphiphilic molecule, particularly TPGS in the nanogel coating does not prevent bioactive agent, therapeutic molecule or drug pharmacological effect.

A biomaterial implant may be any implantable foreign material for clinical use in host mammals such as for prosthetic joints, pacemakers, implantable cardioverter-defibrillators, catheters including intravascular or urinary catheters or materials, stents including coronary stents, mechanical and biological prosthetic heart valves, intraocular lens, dental implants and the like.

A medical device may be, but is not limited to, any device, tool, instrument, implant, or the like, relating to medical field or the practice of human or veterinary medicine, or intended for use to prevent or treat a disease. A medical device may include all natural and synthetic materials and both fibrous and non-fibrous materials. For example, the materials may be comprised of a metal, plastic, paper, glass, ceramic, textile, rubber, polymer, composite material or any other material or combination of materials. Exemplary medical devices include, but are not limited to, any kind of catheter; cannulae; needles; stents of any size, shape, or placement; coils of any size, shape, or placement; contact lenses; Intrauterine devices (IUDS); peristaltic pump chambers; endotracheal tubes; gastroenteric feeding tubes; arteriovenous shunts; condoms; oxygenator and kidney membranes; gloves; pacemaker leads; wound dressings; metallic pins, plates and screws; metallic artificial hips; artificial knees; and gels. In an embodiment, the nanogel of the invention may be used to coat a catheter to prevent bacterial infections.

A bioprosthesis may be, but is not limited to, a prosthesis made of biological material. Examples include heart valves, pericardium, vascular grafts, urinary bladder prostheses, tendon prostheses, hernia patches, surgical mesh and skin substitutes.

In an embodiment, the nanogel of the invention may be used to coat a bioprosthetic heart valve, for example a decellularized porcine heart valve or a bovine pericardium; to prevent bacterial infections and thrombosis.

The coated biomaterial implant, medical device or bioprosthesis may be used in human or animal host for diagnostic, to prevent or treat disease or for medical practice.

In a preferred embodiment the polymer bearing primary or secondary amine groups is poly-(allylamine hydrochloride) of formula (2) wherein p is an integer >1 preferably between 10 and 300.

In another preferred embodiment the bioactive molecule, therapeutic molecule or drug is a triazolo(4,4-d)-pyrimidine derivative of formula (3); preferably
(1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called Triafluocyl or ticagrelor; or
is (1S,2R,3S,4R)-4-[7-[[(1R,2S)-2-(3,4-Difluorophenyl)-cyclopropyl]amino]-5-(propylthio)-3H-1,2,3-triazolo[4,5-d]pyrimidin-3-yl]-1,2,3-cyclopentanetriol also called Fluometacyl that have both anti-bacterial and antiplatelet properties.

In another preferred embodiment the triazolo(4,4-d)-pyrimidine derivative is combined with a bacteriostatic antibiotic, preferably minocycline. Most preferably the triazolo(4,4-d)-pyrimidine derivative is Triafluocyl or Fluometacyl.

The nanogel according to the invention may be anchored or attached onto the surface of the biomaterial implant, medical device or bioprosthesis using various physical or chemical methods known in the art. It is for example electrografting, layer-by-layer deposition, spin-coating, spraying or simply dipping the surface of biomaterial implant, medical device or bioprosthesis into a mixed solution of poly(methacrylamide)-bearing quinone groups of formula (1) with a mixture of bioactive molecule, therapeutic molecule or drug together with an amphiphilic molecule, preferably a vitamin-E derivative.

Alternatively dipping the surface of biomaterial implant, medical device or bioprosthesis into a mixed solution of poly(vinylquinone) with a mixture of one or more bioactive molecule, therapeutic molecule or drug together with an amphiphilic molecule, preferably a vitamin-E derivative is also possible.

The bioactive molecule, therapeutic molecule or drug is loaded into the nanogel in an encapsulated form with the amphiphilic molecule, preferably TPGS and is progressivelly and continuously release over time from the biomaterial implant, medical device or bioprosthesis into mammal host during weeks, preferably 2 weeks. The mammal host may be a human patient or an animal.

When the micelles are loaded into the nanogel, the amphiphilic molecule, particularly TGPS surprisingly reduce bacterial adhesion on the surface and does not prevent the pharmacological effect of the bioactive agent, therapeutic molecule or drug.

Such anti-adhesion effect is synergistically enhanced when a bacteriostatic agent is present into the nanogel.

**In a fourth aspect,** the present invention provides a method of producing a medical device, a biomaterial implant or a bioprosthesis with a coated surface comprising the steps of:
i)dipping the surface to be coated in a buffer solution of dopamine;
ii)dipping the surface coated at step i) into a solution of polymer bearing primary or secondary amine groups; then
iii)dipping the resulting coated surface obtained at step ii) into a mixture of a poly(methacrylamide)-bearing quinone groups of formula (1)

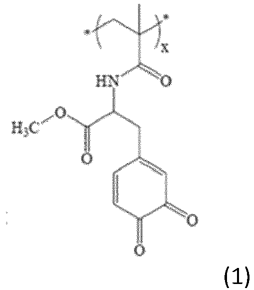

wherein x is an integer > 1, preferably in the range between 1 and 100
with one or more bioactive molecule, therapeutic molecule or drug together with an amphiphilic molecule, preferably a vitamin-E derivative;
lv)drying the crosslinked coated surface obtained at step (iii) to obtain a coated crosslinked nanogel surface comprising one or more bioactive molecule, therapeutic molecule or drug together with the amphiphilic molecule, preferably the vitamin-E derivative.
v)optionally repeating steps ii) to iv) to obtain a surface coated with several layers of crosslinked nanogels comprising one or more bioactive molecule, therapeutic molecule or drug together with the amphiphilic molecule, preferably the vitamin-E derivative.

In step i), the medical device, biomaterial implant or bioprosthesis is first immersed in a buffer solution, particularly a Tris buffer solution with dopamine to strongly anchor the first layer of the nanogel coating at the surface of the medical device, biomaterial implant or bioprosthesis. A primer coating of polydopamine PDA is then generated at the surface of the medical device, by polymerization of dopamine molecule having a 4-(2-aminoethyl) benzene-1,2-diol motif.

In step ii) the polymer bearing primary or secondary amine groups is preferably PAH and covalent grafting of PAH on the primer coating occurs through amine/quinone reaction and/or Schiff base formation.

In step iii) the precoated surface obtained at step ii) is dipped into a solution containing a poly(methacrylamide)-bearing quinone groups of formula (1)
mixed with one or more bioactive molecule, therapeutic molecule or drug together with an amphiphilic molecule, preferably a vitamin-E derivative. Covalent grafting of nanogels is performed through the same reaction and/or Schiff base formation between primary or secondary amines, preferably from PAH monolayer and the quinone groups of poly(methacrylamide of formula (1) 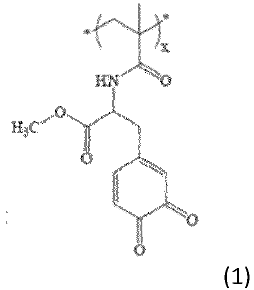 wherein x is an integer > 1, preferably x is between 1 and 100;

In step ii) to v), covalent grafting with the polymer bearing primary or secondary amine groups, preferably PAH occurs between nanogel layers coated on medical device, a biomaterial implant or a bioprosthesis resulting in a crosslinked nanogel coating on medical device, biomaterial implant or bioprosthesis

Alternatively, the present invention provides a method of producing a medical device, a biomaterial implant or a bioprosthesis with a coated surface comprising the steps of:
i)dipping the surface to be coated in a buffer solution of dopamine;
ii)dipping the surface coated at step i) into a solution of polymer bearing primary or secondary amine groups; then
iii)dipping the resulting coated surface obtained at step ii) into a mixture of a of poly(vinylquinone) of formula (6) 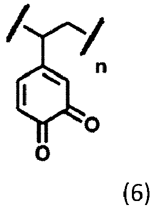 wherein n is an integer >1, preferably between 1 and 100
   with one or more bioactive molecule, therapeutic molecule or drug together with an amphiphilic molecule, preferably a vitamin-E derivative;
iv) drying the crosslinked coated surface obtained at step (iii) to obtain a coated crosslinked nanogel surface comprising one or more bioactive molecule, therapeutic molecule or drug together with the amphiphilic molecule, preferably the vitamin-E derivative.
v) optionally repeating steps ii) to iv) to obtain a surface coated with several layers of crosslinked nanogels comprising one or more bioactive molecule, therapeutic molecule or drug together with the amphiphilic molecule, preferably the vitamin-E derivative.

In step i), the medical device, biomaterial implant or bioprosthesis is first immersed in a buffer solution, particularly a Tris buffer solution with dopamine (DOPA) to strongly anchor the first layer of the nanogel coating at the surface of the medical device, biomaterial implant or bioprosthesis. A primer coating of PDA is then generated at the surface of the medical device, by polymerization of dopamine molecule having 4-(2-aminoethyl) benzene-1,2-diol motif.

In step ii) the polymer bearing primary or secondary amine groups is PAH and covalent grafting of PAH on the primer coating occurs through amine/quinone reaction and/or Schiff base formation at room temperature.

In step iii) the precoated surface obtained at step ii) is dipped into a solution containing a poly(vinylquinone) of formula (6) mixed with one or more bioactive molecule, therapeutic molecule or drug together with an amphiphilic molecule, preferably a vitamin-E derivative. Covalent grafting of nanogels was performed through the same reaction and/or Schiff base formation between primary amines of PAH monolayer and the quinone groups of poly(vinylquinone) of formula (6).

In step ii) to v), covalent grafting with the polymer bearing primary or secondary amine groups, preferably PAH occurs between nanogel layers coated on medical device, biomaterial implant or bioprosthesis resulting in a crosslinked nanogel coating on medical device, a biomaterial implant or a bioprosthesis.

Alternatively, the present invention also provides a method of producing a medical device, a biomaterial implant or a bioprosthesis with a coated surface comprising nanogel made of poly-(methacrylamide)-bearing quinone of formula (1) and polyvinylquinone of formula (6) or the copolymer thereof.

The present invention also extends to methods of producing a biomaterial implant, medical device or bioprosthesis with a surface coated with nanogel made of polymers or copolymers bearing quinone groups.

The obtained nanogel may comprise one or more, preferably two or more bioactive molecules, therapeutic molecules and/or drugs. The bioactive molecules may include an antibiotic and/or an anti-platelet agent.

A medical device, a biomaterial implant or a bioprosthesis with a surface coated with a nanogel comprising two or more layers of nanoparticles may be produced by repeating steps ii) and iii) of the above-described method a) and by repeating steps i) and ii) of method b). A medical device, a biomaterial implant or a bioprosthesis comprising 2, 3, 4, 5 or more layers of nanogel may be produced.

The method of the invention may be used without the need of a primer coating step in method b). In this case, coating adhesion is based on adhesive properties of the free quinone groups present at the nanogel surface, which is sufficient to coat and immobilized the nanogel according to the invention at the surface of the medical device, biomaterial implant or bioprosthesis.

When no primer coating step is used, method b) of producing a medical device, a biomaterial implant or a bioprosthesis with a coated surface is applied comprising the steps of
i)dipping the surface to be coated into a solution of a poly(methacrylamide)-bearing quinone groups of formula (1) mixed with one or more bioactive molecule, therapeutic molecule or drug together with an amphiphilic molecule, preferably a vitamin-E derivative;
ii) dipping the surface coated at step i) into a solution of polymer bearing primary or secondary amine groups;
iii) drying the crosslinked coated surface obtained at step(ii) to obtain a coated crosslinked nanogel surface comprising one or more bioactive molecule, therapeutic molecule or drug together with the amphiphilic molecule, preferably the vitamin-E derivative.
iv)optionally repeating steps i) to iii) to obtain a surface coated with several layers of crosslinked nanogels comprising one or more bioactive molecule, therapeutic molecule or drug together with the amphiphilic molecule, preferably the vitamin-E derivative.

In step ii) to iv), covalent grafting with the polymer bearing primary or secondary amine groups, preferably PAH occurs between nanogel layers coated on medical device, biomaterial implant or bioprosthesis resulting in a crosslinked nanogel coating on medical device, a biomaterial implant or a bioprosthesis. Alternatively, the method of producing a medical device, a biomaterial implant or a bioprosthesis with a coated surface comprises the steps of:
i)dipping the surface to be coated into a solution of a poly(vinyl quinone) of formula (6) mixed with one or more bioactive molecule, therapeutic molecule or drug together with an amphiphilic molecule, preferably a vitamin-E derivative;
ii) dipping the surface coated at step i) into a solution of polymer bearing primary or secondary amine groups; then
iii) drying the crosslinked coated surface obtained at step(ii) to obtain a coated crosslinked nanogel surface comprising one or more bioactive molecule, therapeutic molecule or drug together with the amphiphilic molecule, preferably the vitamin-E derivative.
iv)optionally repeating steps i) to iii) to obtain a surface coated with several layers of crosslinked nanogels comprising one or more bioactive molecule, therapeutic molecule or drug together with the amphiphilic molecule, preferably the vitamin-E derivative.

In a preferred embodiment, the bioactive molecule, therapeutic molecule or drug is a triazolo(4,4-d)-pyrimidine derivative of formula (3); preferably
(1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called Triafluocyl or ticagrelor; or
is (1S,2R,3S,4R)-4-[7-[[(1R,2S)-2-(3,4-Difluorophenyl)-cyclopropyl]amino]-5-(propylthio)-3H-1,2,3-triazolo[4,5-d]pyrimidin-3-yl]-1,2,3-cyclopentanetriol also called Fluometacyl that have both anti-bacterial and antiplatelet properties.

In another preferred embodiment the triazolo(4,4-d)-pyrimidine derivative is combined with a bacteriostatic antibiotic, preferably minocycline. The triazolo(4,4-d)-pyrimidine derivative is preferably Triafluocyl or Fluometacyl.

The method of the invention may be used to coat just a part of the surface of a medical device, a biomaterial implant or a bioprosthesis, or substantially the whole or the whole surface of a medical device, a biomaterial implant or a bioprosthesis.

The invention further provides a coated medical device, a biomaterial implant or a bioprosthesis according to the invention or produced by the method of the invention for use in the prevention or reduction of infection when the medical device, a biomaterial implant or a bioprosthesis is implanted in a mammal that can be a human patient or an animal.

The invention will be further described by means of non-limiting examples only, with reference to the following figures and experimental examples.
**Figure 1** shows dynamic size analysis (dynamic light scattering, DLS) of nanogels particles NTT comprising Triafluocyl and TPGS according to the invention compared to nanogels particles NT comprising triafluocyl only according to WO2018/122318.
**Figure 2** shows nanogels comprising triafluocyl alone (A) or in presence of TPGS (B) according to the present invention. Solid line: DLS analysis realized 1h after nanogel formation. Dashed line: DLS analysis realized after 24h.
**Figure 3** shows surface topography, by a SEM analysis, of a Polyurethane (PU) catheter coated with a nanogel according to the invention (NTT) compared to surface topography of a PU catheter coated with a nanogel (NT) according to WO2018/122318
**Figure 4** shows kinetic release of Triafluocyl from a nanogel (NTT) according to the invention compared to the kinetic release of Triafluocyl from a nanogel (NT) according to WO2018/122318.
**Figure 5** shows nanogels comprising triafluocyl, TPGS and minocycline. Solid line: DLS analysis realized 1h after nanogel formation. Dashed line: DLS analysis realized after 24h.
**Figure 6** shows a microcalorimetric measurement of S. *aureus* BAA-1556 metabolic activity in ethanol solution in the presence of triafluocyl alone or in a mixture with TPGS. Bacterial metabolic activity reflects bacterial growth in solution.
**Figure 7** shows a microcalorimetric measurement of S. *aureus* BAA-1556 metabolic activity adhered on catheters illustrating an anti-attachment property of nanogel coating with triafluocyl alone and together with TPGS for *S*. *aureus. A* delay of peak of bacterial metabolic activity reflects less bacterial attachment on catheter surface.

### Example 1: preparation of the nanogel according to the invention with P(mDOPA) Triafluocyl and TPGS (also called NTT)

The nanogel preparation is similar to the one disclosed in WO2018/122318A1, excepted for the drug loading.

### 1.1 oxidation of PmDOPA → Pox(mDOPA).

A homopolymer of methacrylamide bearing 3,4-dihydroxy-L-phenylalanine (P(mDOPA) is synthetised according to Faure & al in Adv Funct. Mater. 2012; 22:5271-5282, and is oxidized in aqueous media under basic conditions for 12 hours to form hydrosoluble Pox(mDOPA). Oxidized catechol moieties of Pox(mDOPA) are necessary for the covalent interaction of PAH through amine/quinone reaction and/or Schiff base formation at room temperature, and consequently for the preparation of stable cross-linked nanogel.

P(mDOPA) (2.5 mg) was dissolved in distilled water (5 mL) and NaOH (0.1 M) was slowly added in order to raise the pH above 10 and to promote the oxidation of catechol groups of P(mDOPA).

### 1.2 preparation of Triafluocyl/ TPGS mixture in ethanol

Triafluocyl (also called Ticagrelor and provided by Polpharma) and TPGS provided by MedChemExpress LLC were dissolved separately in ethanol to prepare stocks solution of 3,33 mg/mL. 160 µL of triafluocyl solution and 160 µl of TPGS solution were mixed under stirring (300 rpm) with a magnetic stirrer and then concentrated to about 160µl under vacuum at RT.

### 1.3 preparation of the nanogel with Triafluocyl/ TPGS and PAH

Pox(mDOPA) (5ml, 0.5mg/ml) was added under stirring (300 rpm) with a magnetic stirrer, to the concentrated mixture obtained at point 1.2. After one hour of homogenization at RT, an aqueous solution of PAH provided from Sigma Aldrich(0.5 mL, 0.5 mg/ml) at pH 10 was slowly added to the mixture solution. The solution was allowed to react for one hour at room temperature under vigorous stirring (500 rpm) with a magnetic stirrer.

Nanogels particles with a diameter ranging from 100nm to 350 nm were observed by Dynamic Light Spectrometry (Zetasizer Advance Pro,Malvern).

### Example 2: preparation of the nanogel according to the invention with Polyvinyl(quinone), Triafluocyl and TPGS

### 2.1 oxidation of Polyvinyl catechol → Polyvinylquinone).

Similarly to P(mDOPA), poly(vinylcatechol) provided by Polykey is oxidized in aqueous media under basic conditions for 12 hours to form watersoluble Polyvinylquinone. Oxidized catechol moieties of Poly(vinylcathechol) are necessary for the covalent interaction of PAH through amine/quinone reaction and/or Schiff base formation at room temperature, and consequently for the preparation of stable cross-linked nanogel.

Poly(vinylcathechol) (10 mg) was dissolved in distilled water (20 mL) and NaOH (0.1 M) was slowly added in order to raise the pH above 10 for 12 hours and to promote the oxidation of catechol groups of Poly(vinylcatechol).

### 2.2 preparation of Triafluocyl/ TPGS mixture in ethanol

Triafluocyl (also called Ticagrelor) and TPGS were dissolved separately in ethanol to prepare stocks solution of 3,33 mg/mL triafluocyl and of TPGS. 160 µL of triafluocyl solution and 160 µl of TPGS solution were mixed under stirring (300 rpm) with a magnetic stirrer and then concentrated to about 160µl under vacuum at RT.

### 2.3 preparation of the nanogel with Triafluocyl/ TPGS and PAH

Poly(vinylquinone) (5ml, 0.5mg/ml) was added under stirring (300 rpm) with a magnetic stirrer to the concentrated mixture obtained at point 2.2. After one hour of homogenization, an aqueous solution of PAH (0.5 mL, 0.5 mg/ml) at pH 10 was slowly added to the mixture solution. The solution was allowed to react for one hour at room temperature under vigorous stirring (500 rpm) with a magnetic stirrer.

Nanogels particles with a diameter ranging from 100nm to 350 nm were observed by Dynamic Light Spectrometry (Zetasizer Advance Pro, Malvern).

### Example 3: preparation of a medical device with a nanogel according to the invention

A polyurethane catheter provided from Carfill (intravascular grade polyurethane tubing 5Fr), was coated according to the method of the invention.

In Step 1: catheters (4.5 cm long) were dipped into a Dopamine Tris buffer solution pH=7,4 from Aldrich (0.2 g L-1) for 5h.

In Step 2: After rinsing twice with 5ml water, the modified catheter substrate was dipped into an aqueous solution of PAH (pH>10) for 1h and rinsed twice with 5ml water.

In step 3: The modified catheter substrate obtained in step 2 was dipped into an aqueous solution of the bioactive molecule-loaded nanogels loaded prepared according to example 1 or 2 for 18h and rinsed twice with 5ml water.

Steps 2 to 3 were repeated to build-up a multilayer assembly of nanogels on the surface of coated device (five times to obtain a five-layer assembly of cross-linked nanogels).

### Example 4: comparison of the nanogel NTT according to the invention to the nanogel NT of WO2018/122318A1

To allow a comparison with the nanogel of the present invention, a nanogel with and without triafluocyl are prepared according to WO2018/122318A1.

### Nanogel Preparation (NG) without Triafluocyl:

P(mDOPA) (2.5 mg) was dissolved in distilled water (5 mL) and NaOH (0.1 M) was slowly added in order to raise the pH above 10 and to promote the oxidation of catechol groups of P(mDOPA). After one night at room temperature, an aqueous solution of PAH (0.5 mL; 0.5 g/L) at pH 10 was slowly added to the solution of Pox(mDOPA). The solution was allowed to react for one hour at room temperature under vigorous stirring (500 rpm with a magnetic stirrer). Nanogels with a diameter ranging from 100nm to 250 nm were observed by Dynamic Light Spectrometry (Zetasizer Advance Pto, Malvern).

### Nanogel preparation with triafluocyl (NT):

P(mDOPA) (2.5 mg) was dissolved in distilled water (5 mL) and NaOH (0.1 M) was slowly added in order to raise the pH above 10 and to promote the oxidation of catechol groups of P(mDOPA). After one night at room temperature, 0.16ml of Triafluocyl solution (3.33mg/ml in DMSO) was added dropwise to the solution of Pox(mDOPA) under stirring (300 rpm with a magnetic stirrer) at room temperature. After one hour of homogenization, an aqueous solution of PAH (0.5 mL; 0.5 g/L) at pH 10 was slowly added to the mixture solution. The solution was allowed to react for one hour at room temperature under vigorous stirring (500rpm with a magnetic stirrer). Nanogels NT with a diameter ranging from 120nm to 750 nm were observed by Dynamic Light Spectrometry (Zetasizer Advance Pro, Malvern).

### 4.1. Particle size and stability

The nanogel particle diameter size according to the invention is compared to the one obtained for the nanogel (NT) of WO2018/122318A1 with and without triafluocyl.

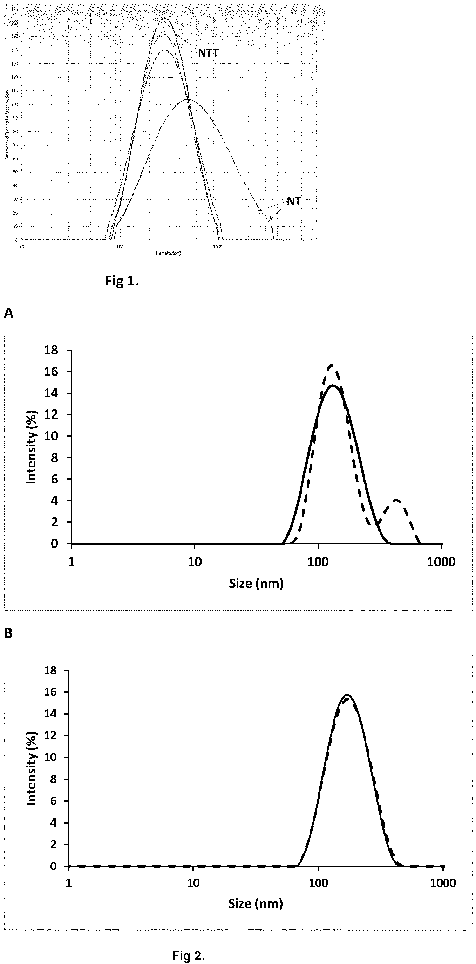

Compared to the nanogel diameter without Triafluocyl and TPGS (ranging from 100nm to 250 nm) and to the nanogel with Triafluocyl only (ranging from 120nm to 750 nm, the present nanogels particles diameter (ranging from 100nm to 350 nm) is clearly intermediate between a nanogel particle alone and a nanogel particle comprising Triafluocyl. The present nanogel invention shows also a lower size distribution as illustrated in figure 1.

Figure 2 shows increased size of NT nanoparticles after 24h (dashed line), depicting nanogel aggregate formation or precipitation, which was not observed for NTT nanoparticles that remained stable after 24h.

Consequently, the nanogel particles according to the invention are more stable than the one disclosed in WO2018/122318A1

### 4.2 Surface topography

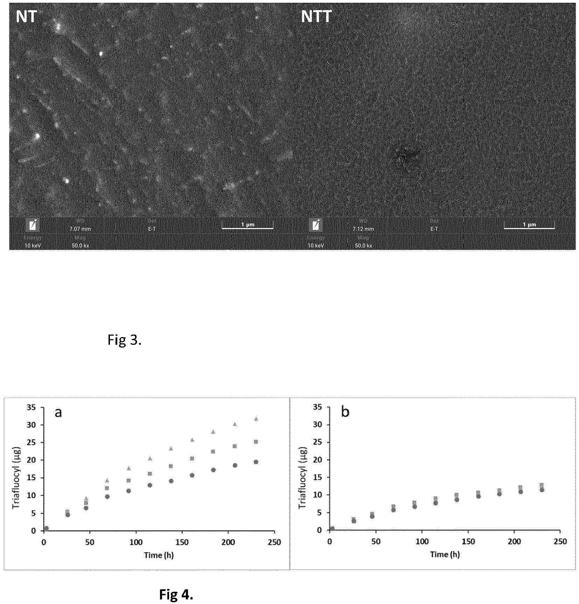

As can be seen from Figure 3, the SEM analysis of the coated catheters showed an obvious difference between NT and NTT coatings. More homogeneous and smooth surface are observed with NTT coating whereas large particle aggregation present in NT coating are observed, which increases surface roughness.

### 4.3 Surface hydrophilicity

Contact angle measurement is a useful method of determining the surface hydrophilicity. Table 1 reports contact angle analysis on glass cover slip (CS) provided by WTR. CS were coated with polydopamine (CS-PDA). one, three and five layers of NTT (CS-NTT 1Layer, 3Layers and SLayers) and compared with five layers of nanogel according to WO2018/122318A1 NT (CS-NT SLayersPEG). As in WO2018/122318A1, PEG (MW 2000 g/mol) was covalently linked on top of nanogel coating. Contact angles were measures after 30 seconds.

The static contact angles decreased obviously in the presence of nanogel layers (NT or NTT) compared to PDA (primer polydopamine). The coated surface with five layers of NTT (nanogel with TPGS) displays much lower water contact angles (18.3°) than the coated surface with same number of layers of NT (35.5°) indicating that the presence of PEG chains (1000g/mol) in TPGS molecules improved the surface hydrophilicity significantly even after the deposition of only one layer of NTT (25.3°).

**Table 1**

| **Contact angle** | **CS-PDA** | **CS-NTT 1Layer** | **CS-NTT 3Layers** | **CS-NTT 5Layers** | **CS-NT 5Layers PEG** |
|---|---|---|---|---|---|
| | 60,3 | 25,3 | 24,6 | 18,3 | 35,5 |

### 4.3 Kinetic release

The *in vitro* drug release from the coated catheters surface was quantified at defined time points up to 10 days. UV spectrophotomet was used to analyze the impact of the presence of TPGS on drug release kinetics and drug release profiles were created for each catheter from the experimentally obtained data.(**Figure 4**)

It was found that the presence of precipitates in NT coating has a significant impact on drug release kinetics of the coated catheters. The drug release data shows that both coatings showed a progressive and continuous release of the drug. However, a large variability was observed for the NT coating, depicting a lack of homogeneity. Moreover, the kinetics of release of triafluocyl with NT was much quicker than with NTT, resulting in shortened release duration, which will negatively impact the duration of coating pharmacological activity.

### 4.4 Triafluocyl content in coated catheters

Six polyurethane 4.5-cm long catheters provided from Carfill (intravascular grade polyurethane tubing 5Fr), were coated with NTT or NT according to the method of the invention (NTT) or to WO2018/122318A1 (NT).

Triafluocyl content in coated catheters (4.5 cm) was determined by HPLC on a Waters Acquity UPLC System consisting of a quaternary solvent delivery system, an injector with adjustable injection volume, temperature controlled autosampler, column thermostat and photo diode array detector. The assay method was used according to ticagrelor monograph (European Pharmacopeia 10.4). Briefly, the analytical column was a XBridge Phenyl, 150x4.6 mm, 3µm (Waters) with the guard column Security Guard Phenyl, 3x4 mm (Phenomenex). An injection volume of 50 µL was used at a flow rate of 1.0 ml/min at 40°C (column temperature). Mobile phase A: was phosphate buffer pH3.0-water-acetonitrile (1:89:10 v/v/v). Mobile phase B: phosphate buffer pH3.0-water-acetonitrile (1:29:70 v/v/v). Detection wavelength was 300 nm.

Data shown in Table 2 indicate that a two-fold higher amount of triafluocyl was obtained in NTT coated catheters as compared to catheters coated with NT.

**Table 2.**

| Sample No. | Coating type | Triaflucocyl content (µg/cm) |
|---|---|---|
| 1 | NT | 6.8 |
| 2 | NT | 5.0 |
| 3 | NT | 5.9 |
| 4 | NT | 4.8 |
| 5 | NT | 6.5 |
| 6 | NT | 5.4 |
| 7 | NTT | 12.0 |
| 8 | NTT | 9.5 |
| 9 | NTT | 11.3 |
| 10 | NTT | 9.8 |
| 11 | NTT | 12.9 |
| 12 | NTT | 11.2 |

### Example 5: preparation of the nanogel according to the invention with P(mDOPA) Triafluocyl, minocycline and TPGS (also called NTTM)

### 5.1 oxidation of PmDOPA → Pox(mDOPA).

A homopolymer of methacrylamide bearing 3,4-dihydroxy-L-phenylalanine (P(mDOPA), is oxidized in aqueous media under basic conditions for 12 hours to form the hydrosoluble Pox(mDOPA). Oxidized catechol moieties of Pox(mDOPA) are necessary for the covalent interaction of PAH through amine/quinone reaction and/or Schiff base formation at room temperature, and consequently for the preparation of stable cross-linked nanogel.

P(mDOPA) (2.5 mg) was dissolved in distilled water (5 mL) and NaOH (0.1 M) was slowly added in order to raise the pH above 10 and to promote the oxidation of catechol groups of P(mDOPA) into Pox(mDOPA).

### 5.2 preparation of Triafluocyl/minocycline/TPGS mixture in ethanol

Triafluocyl (also called Ticagrelor), minocycline and TPGS were dissolved separately in ethanol to prepare stock solutions of 3.33 mg/mL. 160 µL of triafluocyl solution, 320 µl of TPGS solution and 320 µl of minocycline solution were mixed under stirring (300 rpm) and then concentrated to about 160µl under vacuum at RT.

### 5.3 preparation of the nanogel with Triafluocyl/minocycline/TPGS and PAH

Pox(mDOPA) (5ml, 0.5mg/ml) was added under stirring to the concentrated mixture obtained at point 5.2. After one hour of homogenization at RT, an aqueous solution of PAH (0.5 mL, 0.5 mg/ml) at pH 10 was slowly added to the mixture solution. The solution was allowed to react for one hour at room temperature under vigorous stirring (500 rpm).

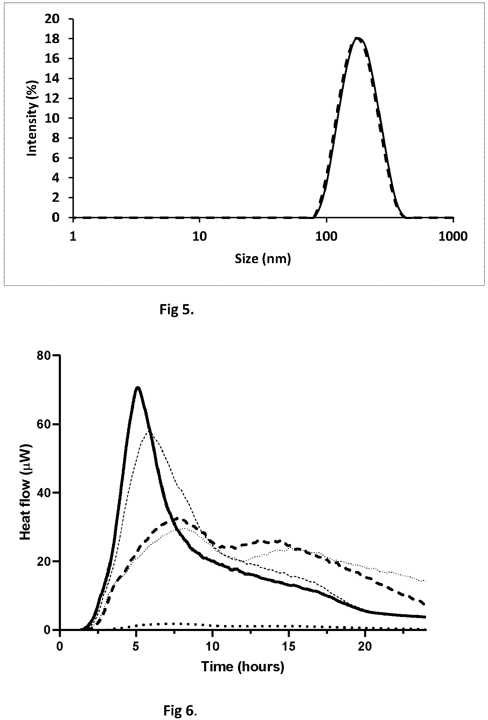

Stable nanogels particles with a diameter ranging from 150nm to 350 nm were observed by Dynamic Light Scattering (Malvern). (**Figure 5**)

NTTM nanogels were stable up to 24h after their formation as were NTT nanogels. In contrast, NT nanogels according to WO2018/122318A1 were not stable (Table 3).

**Table 3**

| Nanogel type | 1h | 3h | 5h | 24h |
|---|---|---|---|---|
| | Particle diameter (nm) | Particle diameter (nm) | Particle diameter (nm) | Particle diameter (nm) |
| NT | 126 | 128 | 132 | 400 |
| NTT | 160 | 160 | 161 | 162 |
| NTTM | 172 | 171 | 171 | 170 |

### Example 6. Effect of TPGS on the inhibitory activity of Triafluocyl on S. aureus biofilm formation.

### 6.1 Effect on planktonic bacterial growth

*S. aureus* (ATCC BAA-1556, MRSA) was grown overnight in TSB (tryptic soy broth) medium, before being diluted 1×10⁴ fold in fresh TSB. Subsequently 300 µL aliquots of diluted bacteria suspensions were supplemented with 10 µg/mL and 20 µg/mL of respectively triafluocyl also called ticagrelor (Polpharma) alone or in the mixture with TPGS (MedChemExpress LLC (Europe)) (1:1 w/w). Both drugs were refrigerated as master stocks prepared in absolute ethanol. Triafluocyl, TPGS or ethanol as a vehicle were added to the bacterial suspensions to obtain the given concentrations followed by a brief vortexing. Bacterial aliquots were then distributed in the dedicated non-activated inserts of a 48-well plate and grown for 24h under static conditions at 37C using Calscreener technology to measure bacterial growth and metabolic activity in Real-Time as described at https://cordis.europa.eu/project/id/784514

***R**eal-time microcalorimetric measurements of bacterial metabolic activity expressed as the heat flow.*

In figure 6, real-time microcalorimetric measurements of S. aureus metabolic activity is reported.

Solid line stands for control bacteria with a vehicle (ethanol); thick dashed line - bacteria treated with 10 µg/mL of triafluocyl; thick dotted line - bacteria treated with 20 µg/mL of triafluocyl; thin dashed line - bacteria treated with both triafluocyl and TPGS (10 µg/mL : 10 µg/mL); thin dotted line - bacteria treated with both triafluocyl and TPGS (20 µg/mL : 20 µg/mL).

Triafluocyl also called ticagrelor significantly reduces S. *aureus* metabolic activity at the concentration of 10 µg/mL, whereas at the higher dose of 20 µg/mL almost no bacterial growth and metabolic activity could form. Strikingly an addition of TPGS at the equivalent amount (w/w) to ticagrelor significantly reduced the inhibitory effect of triafluocyl on bacterial growth and metabolic activity.

### 6 .2 Effect on bacterial attachment in nanogel coating

On the other hand, when triafluocyl is released from the nanogel prepared according to the method of the invention and therefore encapsulated by TPGS, less inhibing effect of TPGS is surprisingly observed as illustrated in the following example.

### Example 7. Testing anti-attachment property of nanogel coating with triafluocyl alone and together with TPGS for S. aureus.

*S. aureus* (ATCC BAA-1556, MRSA) was grown overnight in TSB (tryptic soy broth) medium, before being diluted 1×10⁶ fold in fresh TSB. Subsequently 1000 µL aliquots of diluted bacteria suspensions were supplemented with 2 PU catheter's pieces (0.5 cm length) coated with nanogel loaded either with triafluocyl (0.5 mg/mL) or triafluocyl-TPGS (0.5 mg/mL w/w). PU catheter served as control. Nanogel coating was prepared according to the aforementioned protocol. Bacterial solutions with catheters were incubated for 30 min at 37C and 220 rpm with a magnetic stirrer. Subsequently catheters were washed 2 times with saline (0.9% NaCl) and placed into the dedicated non-activated inserts of a 48-well plate with 300 µL of fresh TSB medium and grown for 24h under static conditions at 37C using Calscreener.

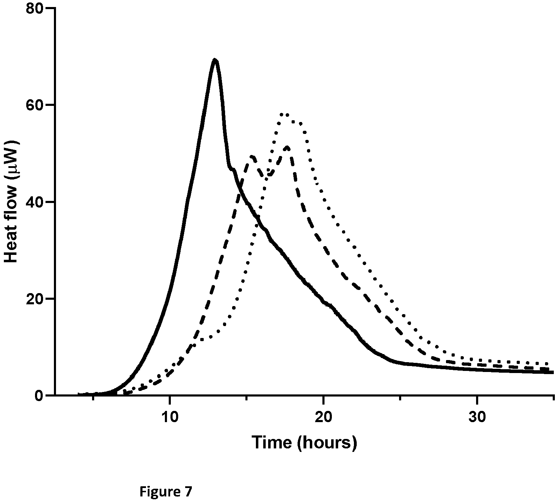

**Figure 7** shows the real-time microcalorimetric measurements of bacterial metabolic activity expressed as the heat flow. Solid line - control PU catheter; dashed line - PU catheter coated with triafluocyl-loaded nanogel; dotted line - PU catheter coated with triafluocyl-TPGS-loaded nanogel.

Nanogel coating with Triafluocyl can increase anti-adhesive property of PU catheter which is seen as the delay of the peak metabolic rate. Presence of TPGS in the coating can possibly further shift the peak of bacterial metabolic activity, depicting less bacterial attachment on catheter surface.

## Claims

1. A nanogel made of a poly(methacrylamide)-bearing quinone groups of formula (1) 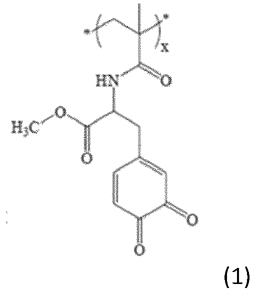
wherein x is an integer > 1, preferably x is between 1 and 100
and/or
made of a poly(vinylquinone) represented by formula (6) 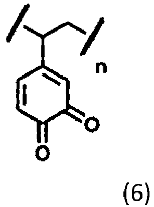
wherein n is an integer > 1, preferably n is between 1 and 100;
and crosslinked with a polymer bearing primary or secondary amine groups; wherein the nanogel comprises one or more bioactive molecule, therapeutic molecule or drug together with an amphiphilic molecule.

2. The nanogel according to claim 1 wherein the amphiphilic molecule is a vitamin-E derivative, preferably D-α-tocophenyl polyethylene glycol succinate.

3. The nanogel according to claim 1 or 2 wherein the polymer bearing primary or secondary amine groups is poly-(allylamine hydrochloride) of formula (2) 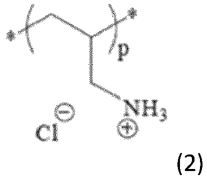 wherein p is an integer >1; preferably p is between 10 and 300.

4. The nanogel according to anyone of claims 1 to 3 wherein the bioactive molecule, therapeutic molecule or drug is a triazolo(4,4-d)-pyrimidine derivative of formula (3) 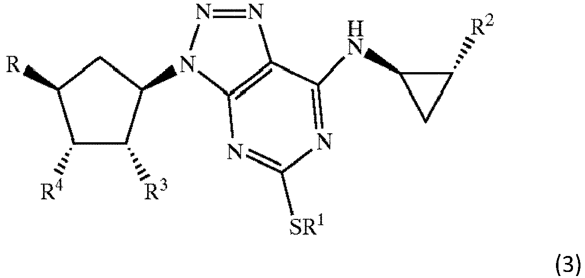
wherein R1 is C3-5 alkyl optionally substituted by one or more halogen atoms; R₂ is a phenyl group, optionally substituted by one or more halogen atoms; R₃ and R₄ are both hydroxyl; R is OH or XOH, wherein X is CH₂, OCH₂CH₂, or a bond;
or a pharmaceutical acceptable salt or solvate thereof, or a solvate thereof or a solvate of such a salt provided that when X is CH₂ or a bond, R₁ is not propyl; when X is CH₂ and R₁ CH₂CH₂CF₃, butyl or pentyl, the phenyl group at R₂ must be substituted by fluorine; when X is OCH₂CH₂ and R₁ is propyl, the phenyl group at R₂ must be substituted by fluorine;
preferably the triazolo (4,4-d)-pyrimidine derivative is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called Triafluocyl; or is (1S,2R,3S,4R)-4-[7-[[(1R,2S)-2-(3,4-Difluorophenyl)-cyclopropyl]amino]-5-(propylthio)-3H-1,2,3-triazolo[4,5-d]pyrimidin-3-yl]-1,2,3-cyclopentanetriol also called Fluometacyl or is a combination thereof

5. The nanogel according to claim 3 wherein the bioactive molecule, therapeutic molecule or drug is a pyrimidine derivative represented by formula (4) 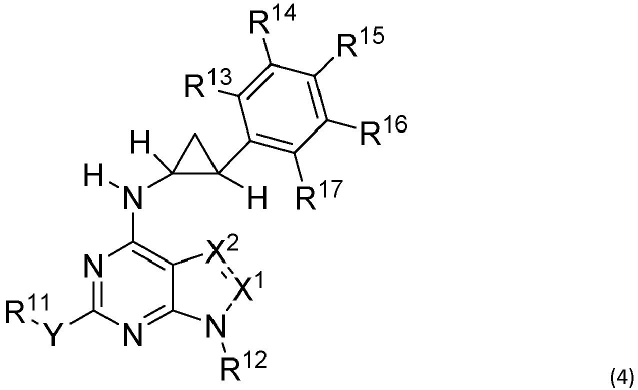
or optical isomers, racemic mixtures thereof, pharmaceutically acceptable acid addition salts, pharmaceutically acceptable metal salts, or alkylated ammonium salts or prodrug thereof;
wherein:
X¹ and X² are independently N, CH, CR⁸ wherein R⁸ is C ₁₋₆ alkyl, C ₂₋₆ alkenyl or C ₂₋₆ alkynyl ; with the exception that if one of X¹ or X² is equal to N, then the remaing X¹ or X² are selected from CH, CR⁸ .
-Y- is -O- or -S-;
R¹¹ and R¹² are independently C₁₋₆ -alkyl, C₂₋₆-alkenyl, C₂₋₆-alkynyl, C₃₋₆-cycloalkyl, aryl, aryl-C₁₋₆-alkyl wherein the alkyl or cycloalkyl moiety is optionally mono or polysubstituted with OH or an halogen and the aryl moiety is optionally mono or polysubstituted with an halogen, -C₁₋₆ alkyl, -C₁₋₆ alkoxy, -OH, -NO₂, -CN, -NH₂, -NHR⁸, -N(R⁸)₂ -COOH, -COOR⁸, -CONH₂, -CONHR⁸, -CON(R⁸)₂, -SO₂NH₂, - SO₂NHR⁸, or -SO₂N(R⁸)₂;
R¹³, R¹⁴, R¹⁵, R¹⁶ and R¹⁷ are independently H, an halogen, a C₁₋₆ alkyl, C₁₋₆ alkoxy, -OH, -NO₂, -CN, -NH₂, -NHR⁸, -N(R⁸)₂ -COOH, -COOR⁸, -CONH₂, -CONHR⁸, - CON(R⁸)₂, -SO₂NH₂, -SO₂NHR⁸, or -SO₂N(R⁸)₂.

6. The nanogel according to any of claims 2 to 5 wherein the bioactive molecule, therapeutic molecule or drug and D-α-tocophenyl polyethylene glycol succinate are in a ratio between 1:1 w/w to 1:5 w/w.

7. The nanogel according to any of claims 1 to 6 wherein the one or more bioactive molecule, therapeutic molecule or drug are an antivirulence or a bactericidal agent together with a bacteriostatic agent, preferably minocycline.

8. A biomaterial implant, medical device or bioprosthesis wherein a surface or part thereof is coated with the nanogel according to any of claims 1 to 7.

9. The biomaterial implant, medical device according to claim 8 wherein the medical device is a catheter.

10. A method of making a nanogel comprising one or more bioactive molecule, therapeutic molecule or drug, the method comprising the steps of:
i) mixing poly(methacrylamide)-bearing quinone groups of formula (1) 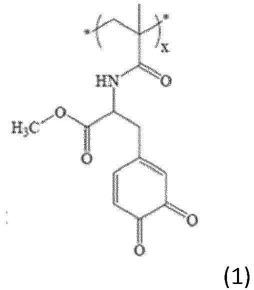
wherein x is an integer > 1, preferably x is between 1 and 100
and/or a poly(vinylquinone) represented by formula (6) 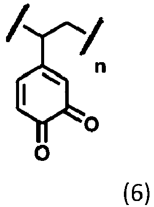
wherein n is an integer > 1, preferably n is between 1 and 100 ;
with a one or more bioactive molecule, therapeutic molecule or drug together with an amphiphilic molecule;
ii) adding a solution of polymer bearing primary or secondary amine groups to the resulting mixture of step i) to generate a nanogel comprising one or more bioactive molecule, therapeutic molecule or drug together with the amphiphilic molecule.

11. The method according to claim 10, wherein the amphiphilic molecule is a vitamin-E derivative, preferably D-α-tocophenyl polyethylene glycol succinate.

12. The method according to claim 11 wherein the ratio of bioactive molecule, therapeutic molecule or drug with D-α-tocophenyl polyethylene glycol succinate is between 1:1 w/w and 1:5 w/w.

13. The method according to any one of claims 10 to 12 wherein the one or more bioactive molecule, therapeutic molecule or drug is a triazolo(4,4-d)-pyrimidine derivative of formula (3); preferably the triazolo(4,4-d)-pyrimidine derivative is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called Triafluocyl; or (1S,2R,3S,4R)-4-[7-[[(1R,2S)-2-(3,4-Difluorophenyl)-cyclopropyl]amino]-5-(propylthio)-3H-1,2,3-triazolo[4,5-d]pyrimidin-3-yl]-1,2,3-cyclopentanetriol also called Fluometacyl or a combination thereof.

14. The method according to claim anyone of claims 10 to 12 wherein bioactive molecule, therapeutic molecule or drug is a pyrimidine derivative represented by formula (4) 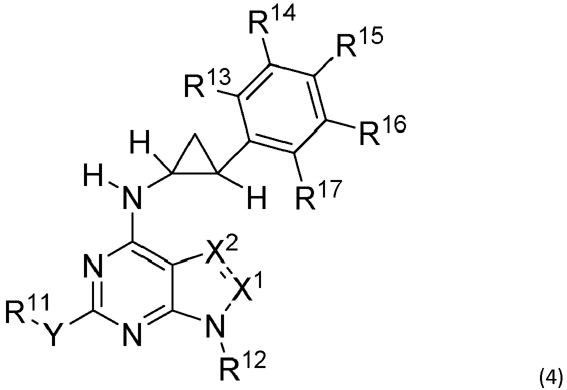
or optical isomers, racemic mixtures thereof, pharmaceutically acceptable acid addition salts, pharmaceutically acceptable metal salts, or alkylated ammonium salts or prodrug thereof;
wherein:
X¹ and X² are independently N, CH, CR⁸ wherein R⁸ is C ₁₋₆ alkyl, C ₂₋₆ alkenyl or C ₂₋₆ alkynyl ; with the exception that if one of X¹ or X² is equal to N, then the remaing X¹ or X² are selected from CH, CR⁸.
-Y- is -O- or -S-;
R¹¹ and R¹² are independently C₁₋₆ -alkyl, C₂₋₆-alkenyl, C₂₋₆-alkynyl, C₃₋₆-cycloalkyl, aryl, aryl-C₁₋₆-alkyl wherein the alkyl or cycloalkyl moiety is optionally mono or polysubstituted with OH or an halogen and the aryl moiety is optionally mono or polysubstituted with an halogen, -C₁₋₆ alkyl, -C₁₋₆ alkoxy, -OH, -NO₂, -CN, -NH₂, -NHR⁸, -N(R⁸)₂ -COOH, -COOR⁸, -CONH₂, -CONHR⁸, -CON(R⁸)₂, -SO₂NH₂, - SO₂NHR⁸, or -SO₂N(R⁸)₂;
R¹³, R¹⁴, R¹⁵, R¹⁶ and R¹⁷ are independently H, an halogen, a C₁₋₆ alkyl, C₁₋₆ alkoxy, -OH, -NO₂, -CN, -NH₂, -NHR⁸, -N(R⁸)₂ -COOH, -COOR⁸, -CONH₂, -CONHR⁸, - CON(R⁸)₂, -SO₂NH₂, -SO₂NHR⁸, or -SO₂N(R⁸)₂.

15. The method according to claim anyone of claims 10 to 14 wherein the one or more bioactive molecule, therapeutic molecule or drug are an antivirulence or a bactericidal agent together with a bacteriostatic agent, preferably minocycline.

16. A method of producing a medical device, a biomaterial implant or a bioprosthesis with a coated surface, wherein the coating is obtained by one of both following coating preparation:
(a)comprising the following in-sequence steps:
i) dipping the surface to be coated in a buffer solution of dopamine;
ii) dipping the surface coated with dopamine at step i); in a solution of polymer bearing primary or secondary amine groups; then
iii) dipping the resulting coated surface obtained at step ii) in a mixture of a poly(methacrylamide)-bearing quinone groups of formula (1) 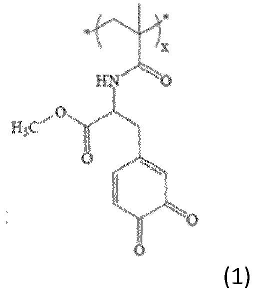
wherein x is an integer > 1, preferably x is between 1 and 100
and/or of a poly(vinylquinone) of formula (6) 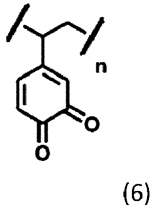
wherein n is an integer > 1, preferably n is between 1 and 100; with one or more bioactive molecule, therapeutic molecule or drug together with an amphiphilic molecule;
iv)drying the crosslinked coated surface obtained at step iii) to obtain a coated crosslinked nanogel surface comprising one or more bioactive molecule, therapeutic molecule or drug together with the amphiphilic molecule;
v)optionally repeating steps ii) to iv) to obtain a surface coated with several layers of crosslinked nanogels comprising one or more bioactive molecule, therapeutic molecule or drug together with the amphiphilic molecule, preferably the vitamin-E derivative.
or (b) comprising the following in-sequence steps:
i) dipping the surface to be coated in a mixture of a poly(methacrylamide)-bearing quinone groups of formula (1) 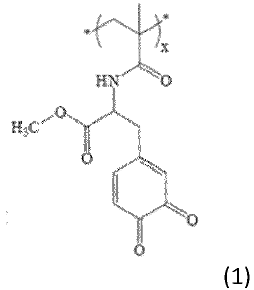
wherein x is an integer > 1, preferably x is between 1 and 100;
and/or of a poly(vinylquinone) of formula (6) 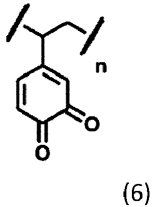
wherein n is an integer > 1, preferably n is between 1 and 100; with one or more bioactive molecule, therapeutic molecule or drug together with an amphiphilic molecule;
ii)dipping the surface coated at step i); in a solution of polymer bearing primary or secondary amine groups; then
iii)drying the crosslinked coated surface obtained at step iii) to obtain a coated crosslinked nanogel surface comprising one or more bioactive molecule, therapeutic molecule or drug together with an amphiphilic molecule.
iv) optionally repeating steps i) to iii) to obtain a surface coated with several layers of crosslinked nanogels comprising one or more bioactive molecule, therapeutic molecule or drug together with the amphiphilic molecule, preferably the vitamin-E derivative.

17. The method according to claim 16 wherein the amphiphilic molecule is a vitamin-E derivative, preferably D-α-tocophenyl polyethylene glycol succinate.

18. The method according to anyone of claims 16 to 17 wherein the one or more bioactive molecule, therapeutic molecule or drug is a triazolo(4,4-d)-pyrimidine derivative or a pyrimidine derivative together with a bacteriostatic agent, preferably minocycline.

19. The method according to anyone of claims 16 to 18 wherein the one or more bioactive molecule, therapeutic molecule or drugs is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called Triafluocyl; or (1S,2R,3S,4R)-4-[7-[[(1R,2S)-2-(3,4-Difluorophenyl)-cyclopropyl]amino]-5-(propylthio)-3H-1,2,3-triazolo[4,5-d]pyrimidin-3-yl]-1,2,3-cyclopentanetriol also called Fluometacyl, or a combination thereof.
